# EUROPÄISCHE PATENTSCHRIFT

(11) **EP 2 574 305 B1**
(45) Veröffentlichungstag und Bekanntmachung des Hinweises auf die Patenterteilung: **26.02.2014**
(21) Anmeldenummer: 12181218.4
(22) Anmeldetag: 21.08.2012
(51) Int. Cl.: A61F 2/06, A61M 1/10

(54) **Ausgleichsgefäß**
Compensation vessel
Tube de compensation

(30) Priorität: 27.09.2011 DE 102011053988
(43) Veröffentlichungstag der Anmeldung: 03.04.2013
(73) Patentinhaber: E.S. Bio-Tech Limited, 3030 Limassol (CY)
(72) Erfinder: Baecke, Martin, Dr., 06847 Dessau-Roßlau (DE); Doss, Mirko, Dr., 60599 Frankfurt (DE)
(74) Vertreter: Albiger, Jonas

(56) Entgegenhaltungen:
- EP-A1- 2 319 454
- WO-A1-89/01765
- DE-A1-102004 018 255
- DE-A1-102005 058 409
- DE-T2- 69 622 757
- US-A1- 2010 204 539

## Beschreibung

Die vorliegende Erfindung betrifft ein Ausgleichsgefäß zur Beeinflussung des Blutdrucks, umfassend eine Volumenkammer mit Anschlussmitteln zum Anschluss der Volumenkammer an ein natürliches kardiovaskuläres System, wobei durch eine Druckänderung in dem kardiovaskulären System eine Volumenänderung der Volumenkammer bewirkbar ist, sowie Anpassungsmittel, welche die Volumenänderung der Volumenkammer in einem unteren Druckbereich unterhalb eines mindestens 100 mmHg betragenden Druckschwellenwertes auf maximal 10 cm³ begrenzen und welche in einem oberen Druckbereich zwischen dem Druckschwellenwert und 150 mmHg eine Volumenänderung der Volumenkammer von mindestens 10 cm³ bewirken.

Als arterielle Hypertonie, kurz Bluthochdruck, bezeichnet man ein Krankheitsbild, bei dem der Blutdruck des arteriellen Gefäßsystems chronisch erhöht ist. Nach Definition der WHO gilt ein dauerhafter systolischer Blutdruck höher als 140 mmHg und ein diastolischer Blutdruck höher als 90 mmHg als Bluthochdruck. Vor allem in den westlichen Industrieländern ist Bluthochdruck weit verbreitet. Als Ursache kommen beispielsweise genetische Veranlagung, Auswirkungen von Medikamenten, Genussmitteln und Drogen sowie andere Grunderkrankungen in Frage. Anhaltender Bluthochdruck führt häufig zu Folgeschäden wie Herzinfarkt und Schlaganfall.

Die Behandlung von Bluthochdruck erfolgt heute zumeist medikamentös in Verbindung mit einer Änderung des Lebensstils. Der Erfolg beider Therapieformen ist jedoch in hohem Maße von der Kooperationsfähigkeit und -bereitschaft des behandelten Patienten abhängig. Insbesondere ältere Menschen sind oft nur schwer in der Lage, ihren Lebensstil zu ändern und eine Medikamenteneinnahme mit der erforderlichen Regelmäßigkeit zu bewerkstelligen. Darüber hinaus wird die regelmäßige Medikamenteneinnahme häufig als lästig und einschränkend empfunden und sie kann eine Belastung für andere Organe des menschlichen Körpers darstellen. Es werden auch Medikamentenunverträglichkeiten beobachtet.

Es sind daher bereits Versuche unternommen worden, Vorrichtungen zur Beeinflussung des Blutdrucks bereitzustellen, durch die insbesondere Bluthochdruck effektiv und möglichst ohne die Notwendigkeit der Einnahme von Medikamenten therapiert werden kann.

Aus der DE 10 2005 058 409 A1 ist ein Implantat als Ersatz eines Abschnitts der Aorta oder einer Arterie bekannt, dessen Volumen durch ein in die Gefäßwandung eingearbeitetes Federmittel elastisch verformbar ausgebildet ist. Durch dieses Implantat sollen Druckänderungen im kardiovaskulären System reduziert werden.

Die WO 89/01765 A1 beschreibt ein Implantat und ein Verfahren zur Steigerung des Blutdurchflusses durch Erhöhung der arteriellen Dehnbarkeit (des arteriellen Volumens) und durch Reduzierung der Druckschwankungen (-pulsation) im arteriellen System sowie zur Verbesserung (Verstärkung) der Durchblutung spezifischer Organe, um schädlichen Effekten kardiovaskulärer Krankheiten entgegenzuwirken.

In der EP 2 319 454 A1 ist ein Ausgleichsgefäß zur Beeinflussung des Blutdrucks gemäß dem Oberbegriff des Patentanspruchs 1 offenbart. Es handelt sich dabei im Wesentlichen um ein Implantat mit einer Volumenkammer mit Verbindungsmitteln zum Verbinden der Volumenkammer mit einem natürlichen kardiovaskulären System und mit Anpassungsmitteln, durch die eine Volumenänderung eines Volumens der Volumenkammer bei einer Druckänderung in dem kardiovaskulären System oder in der Volumenkammer ermöglicht oder bewirkt wird, wobei die Volumenänderung in einem unteren Druckbereich zwischen 50 mmHg und einem mindestens 100 mmHg betragenden Druckschwellenwert höchstens 10 cm³ und in einem oberen Druckbereich zwischen dem Druckschwellenwert und 150 mmHg mindestens 10 cm³ beträgt. Aufgabe der vorliegenden Erfindung ist die Weiterentwicklung eines derartigen Ausgleichsgefäßes.

Diese Aufgabe wird gelöst durch die Merkmale des Patentanspruchs 1.

Vorteilhafte Ausgestaltungen und Weiterbildungen der Erfindung sind Gegenstand der Unteransprüche.

Die Erfindung sieht vor, dass die Anpassungsmittel einen Rahmen sowie mindestens einen mit diesem zusammenwirkenden Federkörper umfassen, wobei Rahmen und Federkörper außerhalb der Volumenkammer angeordnet sind und wobei der Rahmen zwei Stirnteile sowie mindestens drei die beiden Stirnteile verbindende Stützstäbe aufweist, zwischen denen sich der mindestens eine Federkörper erstreckt.

Mit anderen Worten weist das erfindungsgemäße Ausgleichsgefäß eine innenliegende, für den Blutdurchfluss bestimmte Volumenkammer sowie einen äußeren Rahmen und mindestens einen mit diesem zusammenwirkenden Federkörper auf, wobei der Rahmen und der Federkörper gemeinsam als Anpassungsmittel fungieren und das spezielle druckabhängige Volumenverhalten der Volumenkammer bestimmen.

So ist es erfindungsgemäß vorgesehen, dass der die Volumenkammer umgebende Rahmen mit Federkörper die Volumenänderung der Volumenkammer in einem unteren Druckbereich begrenzt, während er ab einem voreinstellbaren Druckschwellenwert plötzlich eine große Volumenänderung bewirkt oder zulässt. Der in den Rahmen eingespannte Federkörper reagiert dabei in einem bevorzugten Ausführungsbeispiel nach Art eines eingespannten Knickstabes, der erst bei Überschreiten einer kritischen Knicklast plötzlich seitlich ausweicht. Bei dem erfindungsgemäßen Ausgleichsgefäß übt entsprechend der Federkörper einen solchen Druck von außen auf die Volumenkammer aus, dass in einem unteren Blutdruckbereich nur minimale Volumenänderungen der Volumenkammer zugelassen werden. Oberhalb des Druckschwellenwertes hingegen kann der Federkörper dem von innen auf die Wand der Volumenkammer wirkenden Blutdruck nicht standhalten und weicht sprunghaft nach außen aus. Hierdurch vergrößert sich das Volumen der Volumenkammer schlagartig, so dass der Blutdruck absinkt.

Das spezielle druckabhängige Verhalten des Federkörpers wird dabei unter anderem durch das Zusammenwirken des Federkörpers mit dem als Widerlager dienenden Rahmen bestimmt, welcher erfindungsgemäß aus zwei Stirnteilen und mindestens drei die Stirnteile verbindenden Stützstäben besteht. In den von den Stirnteilen und den Stützstäben aufgespannten festen Rahmen ist der Federkörper so eingespannt, dass er abhängig von den in der Volumenkammer herrschenden Blutdruckverhältnissen sprunghaft von einer eine Volumenänderung der Volumenkammer begrenzenden Stellung in eine eine deutliche Volumenänderung der Volumenkammer zulassende Stellung übergehen kann. Umgekehrt geht der Federkörper bei nachlassendem Blutdruck im Bereich des Druckschwellenwertes wieder in die eine Volumenänderung der Volumenkammer begrenzende Stellung über. Als Material für die Stützstäbe sowie für die Stirnteile kommen vorzugsweise feste Materialien wie Stahl, Titan oder verstärkte Kunststoffe in Frage.

Der Anschluss der Volumenkammer an natürliche Blutgefäße, insbesondere im Bereich der Aorta, erfolgt bevorzugt durch Vernähen im Bereich der Anschlussmittel in an sich bekannter Weise. Eine zusätzliche Fixierung, gegebenenfalls auch an umliegendem Körpergewebe, kann mit Hilfe des Rahmens erfolgen, der zu diesem Zweck im Bereich der Stirnteile mit Löchern oder Laschen für die Durchführung von Fäden versehen sein kann. Dabei kann das erfindungsgemäße Ausgleichsgefäß grundsätzlich sowohl parallel nach Art eines Bypasses als auch in Reihe zu einem körpereigenen Blutgefäß eingesetzt werden.

Die Volumenkammer ist vorzugsweise als Gewebe- bzw. Gewirkschlauch aus Polyester gefertigt. Polyestergewebe/-gewirk ist zugfest und biegeschlaff und hat sich als Material für Aorta-Implantate bewährt. Alternativ können auch andere als Aorta-Implantate geeignete Materialien verwendet werden. Die von Blut durchströmte Volumenkammer erfährt in Abhängigkeit von den herrschenden Blutdruckverhältnissen eine Formänderung, wodurch sie in der Lage ist, den natürlichen Windkesseleffekt nachzubilden. Als Windkesseleffekt bezeichnet man das Zurückhalten eines Teils des vom Herzen ausgeworfenen Blutvolumens während der Systole in den elastischen Arterien und dessen kontinuierliche Abgabe während der Diastole, wodurch der Blutstrom vergleichmäßigt wird. Diese den Windkesseleffekt bestimmenden minimalen Volumenänderungen der Volumenkammer in einem unteren Druckbereich, welche denen natürlicher Gefäße nachgebildet sind, werden durch die erfindungsgemäßen Anpassungsmittel zugelassen, während große Volumenänderungen der Volumenkammer erfindungsgemäß erst bei Überschreiten eines kritischen Druckschwellenwertes bewirkt werden.

Gemäß einer Ausgestaltung der Erfindung weisen die Stirnteile Halterungen für die Aufnahme der Stützstäbe auf, in welchen die Stützstäbe verdrehsicher gelagert sind. Ein weiterer Vorschlag sieht vor, dass der Federkörper Durchführungen für die Stützstäbe aufweist, wobei die Durchführungen vorzugsweise ebenfalls verdrehsicher mit den Stützstäben verbunden sind. Durch die verdrehsichere Verbindung der Stützstäbe mit den Halterungen sowie insbesondere der Stützstäbe mit den Durchführungen in dem Federkörper wird zum einen verhindert, dass das Material des Federkörpers, der Stützstäbe sowie der Halterungen durch permanentes Verdrehen und die damit verbundene Reibung übermäßig belastet und dadurch einem schnellen Verschleiß ausgesetzt wird. Zum anderen wird durch die verdrehsichere Lagerung sichergestellt, dass der Federkörper bei in der Volumenkammer allmählich ansteigendem Blutdruck nicht bereits durch leichtes Verdrehen gegenüber den Stützstäben graduell nach außen ausweicht. Vielmehr ist der Federkörper gemäß dieser Ausgestaltung der Erfindung im Bereich der Durchführungen gegenüber den Stützstäben vollkommen fixiert, wodurch der Effekt des abrupten Übergangs des Federkörpers von der volumenbegrenzenden in die volumenfreigebende Stellung verstärkt wird. Gleichzeitig wird dadurch ein Einsetzen der Rückstellbewegung bei relativ hohem Druck ermöglicht.

Es hat sich gezeigt, dass ein flächig ausgeführter Federkörper aus Silikon das gewünschte Federverhalten aufweist. Alternativ kann der Federkörper beispielsweise aus Polyurethan gefertigt sein. Durch unterschiedliche Dimensionierung, Materialstärke bzw. eine Profilierung des verwendeten Materials kann das gewünschte Federverhalten, d.h. insbesondere der Druckschwellenwert sowie die Volumenzunahme im oberen Druckbereich, vorab eingestellt und an die individuellen Bedürfnisse eines zu behandelnden Patienten angepasst werden.

Gemäß einer Ausgestaltung der Erfindung liegt der Federkörper zumindest abschnittsweise an der Außenwand der Volumenkammer an. Durch diesen direkten Kontakt zu der Volumenkammer wird der durch den Blutdruck bewirkte Druck auf die Wand der Volumenkammer unmittelbar auf den Federkörper übertragen.

Bei einer bevorzugten Ausführung der Erfindung weist der Rahmen vier die beiden Stirnteile verbindende Stützstäbe sowie der Federkörper vier Federflächen auf, wobei sich jeweils eine Federfläche zwischen zwei benachbarten Stützstäben erstreckt. Der Rahmen erhält so eine quaderförmige Gestalt, wobei die vier Stützstäbe die Längskanten und die vier Federflächen federnde Seitenflächen des Quaders bilden.

Ein weiterer Vorschlag sieht vor, dass in dem unteren Druckbereich zwei einander gegenüberliegende Federflächen des Federkörpers eine konkave Krümmung aufweisen und die beiden übrigen Federflächen eine konvexe Krümmung aufweisen, während in dem oberen Druckbereich alle vier Federflächen des Federkörpers eine konvexe Krümmung aufweisen. Die wesentliche Federwirkung des Federkörpers geht somit von den beiden im unteren Druckbereich konkav, d.h. auf die Volumenkammer zu gekrümmten Federflächen aus, welche ihr Krümmungsverhalten oberhalb des Druckschwellenwertes sprunghaft ändern und nach außen ausweichen. Durch die konkave Krümmung zweier Federflächen im unteren Druckbereich kann die elastische Volumenkammer knochenförmig verformt werden. Erst bei Blutdrücken oberhalb des Druckschwellenwertes ist der Druck auf die Wand der Volumenkammer und damit auf die dort anliegenden Federflächen so groß, dass letztere sprunghaft nach außen federn und sich der knochenförmig verformte Querschnitt der Volumenkammer zu einem kreisförmigen bzw. elliptischen Querschnitt aufweitet. Der mit dieser Aufweitung verbundene Volumenzuwachs der Volumenkammer führt zu dem gewünschten Absinken des Blutdrucks.

Das Verhältnis von Länge zu Breite zu Höhe eines erfindungsgemäßen Ausgleichsgefäß beträgt typischerweise etwa 1:0,4:0,5. So liegt beispielsweise bei einer Länge des Ausgleichgefäßes in einem Bereich zwischen 90 mm und 130 mm die Breite vorzugsweise in einem Bereich zwischen 36 mm und 52 mm und die Höhe in einem Bereich zwischen 45 mm und 65 mm.

Im Folgenden wird die Erfindung anhand eines Ausführungsbeispiels und unter Bezugnahme auf die beigefügten Zeichnungen näher erläutert. Es zeigen:
- Figur 1: Ein erfindungsgemäßes Ausgleichsgefäß in perspektivischer Ansicht;
- Figur 2: Eine Seitenansicht eines erfindungsgemäßen Ausgleichsgefäßes;
- Figur 3: Eine Draufsicht von oben auf ein erfindungsgemäßes Ausgleichsgefäß;
- Figur 4: Eine Draufsicht von vorne auf ein erfindungsgemäßes Ausgleichsgefäß.

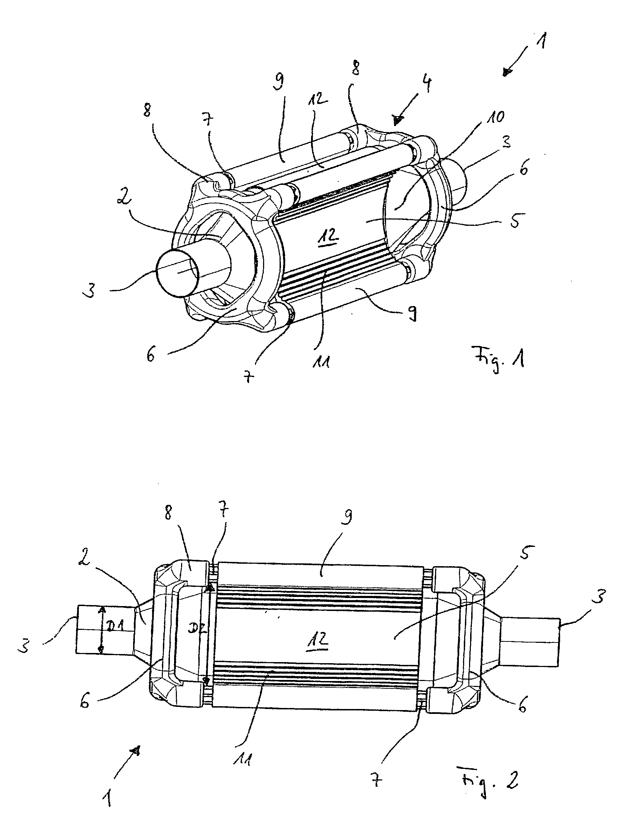

Die Figuren 1 bis 4 zeigen ein im Ganzen mit 1 bezeichnetes erfindungsgemäßes Ausgleichsgefäß im noch nicht an ein natürliches Blutgefäß angeschlossenen Zustand. Es umfasst eine innenliegende, längliche Volumenkammer 2 aus Polyestergewebe, sowie einen außerhalb der Volumenkammer 2 angeordneten Rahmen 4. Die Volumenkammer 2 weist an ihren beiden Enden Anschlussmittel 3 für den Anschluss an ein natürliches Blutgefäß auf, wobei die Volumenkammer 2 im Allgemeinen im Bereich der Anschlussmittel 3 mit dem natürlichen Blutgefäß vernäht wird. Die Anschlussmittel 3 weisen einen Durchmesser D1 auf, während sich die Volumenkammer 2 in an die Anschlussmittel 3 anschließenden Übergangsbereichen allmählich bis auf einen Durchmesser D2 aufweitet, vgl. Figur 2. Die tatsächlichen Werte der Durchmesser D1 und D2 hängen unter anderem davon ab, ob das Ausgleichsgefäß parallel nach Art eines Bypasses oder in Reihe zu einem körpereigenen Blutgefäß eingesetzt wird. In einer Bypass-Anordnung, bei der lediglich ein Teilvolumen des Blutes durch das Ausgleichsgefäß 1 strömt, liegt der Durchmesser D1 beispielsweise in einem Bereich zwischen 5 mm und 20 mm, vorzugsweise zwischen 10 mm und 15 mm. Wird das Ausgleichsgefäß 1 hingegen in Reihe angeordnet, so müssen die Anschlussmittel 3 und gegebenenfalls auch die Volumenkammer 2 selbst größer gewählt werden. Der Durchmesser D1 liegt in diesem Fall typischerweise in einem Bereich zwischen 20 mm und 40 mm, vorzugsweise zwischen 25 mm und 35 mm.

Der die Volumenkammer 2 umgebende Rahmen 4 besteht aus zwei im Wesentlichen oval bis ringförmig ausgebildeten Stirnteilen 6 sowie vier die beiden Stirnteile 6 verbindenden Stützstäben 7, wobei der Rahmen 4 eine etwas geringere Länge aufweisen kann als die Volumenkammer 2. Die Volumenkammer 2 wird derart in den Rahmen 4 eingeführt, dass die beiden Stirnteile 6 in den oben im Zusammenhang mit der Durchmesseraufweitung der Volumenkammer 2 beschriebenen Übergangsbereichen zu liegen kommen und die Anschlussmittel 3 durch zentrale Öffnungen in den Stirnteilen 6 aus dem Rahmen 4 herausragen, siehe Figuren 1 und 2. Die Stirnteile 6 können darüber hinaus mit in den Figuren nicht dargestellten Laschen und/oder Löchern ausgebildet sein, welche neben den Anschlussmittein 3 zusätzliche Möglichkeiten für die Fixierung des Ausgleichsgefäßes 1 an einem natürlichen Blutgefäß oder umliegendem Bindegewebe bieten.

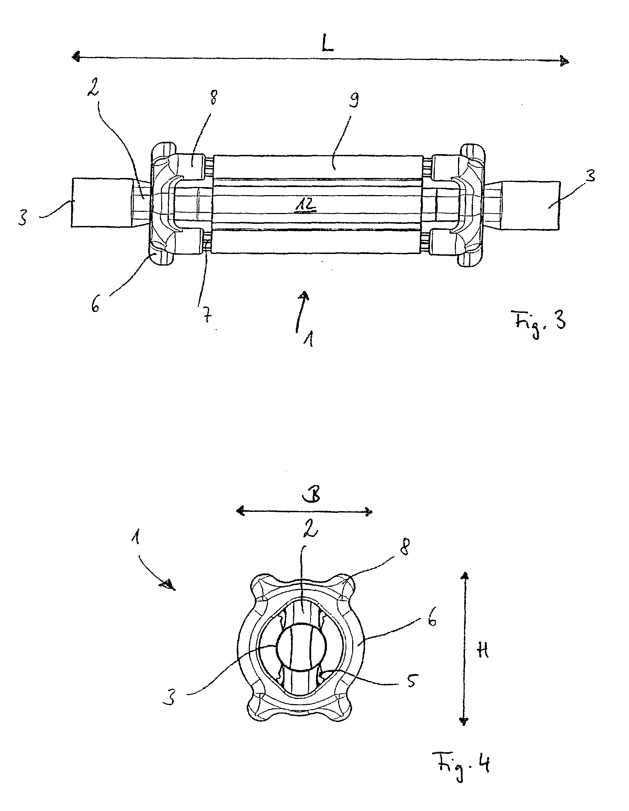

Jedes der beiden Stirnteile 6 weist vier angeformte Halterungen 8 für die Aufnahme der Stützstäbe 7 auf. Wie insbesondere aus Figur 4 ersichtlich ist, sind die Halterungen 8 dabei zwar symmetrisch, jedoch derart angeordnet, dass gemäß der Darstellung in Figur 4 die beiden oberen Halterungen bzw. die beiden unteren Halterungen jeweils einen kleineren Abstand voneinander aufweisen als die linke obere und die linke untere Halterung bzw. die rechte obere und die rechte untere Halterung. Durch diese Anordnung erhält das Ausgleichsgefäß 1 eine insgesamt eher flache Gestalt, was das Einführen und Positionieren in einem menschlichen Körper erleichtert.

Mit Hilfe der vier in die Halterungen 8 der beiden Stirnteile 6 eingeführten Stützstäbe 7 wird der Rahmen 4 komplett aufgespannt. Die Stützstäbe 7 sind mit einem Profil versehen, mit welchem sie in ein korrespondierendes Innenprofil der Halterungen 8 eingreifen, so dass die Stützstäbe 7 verdrehsicher in den Halterungen 8 gelagert sind.

Zwischen den Stützstäben 7 erstreckt sich ein einstückig ausgebildeter Federkörper 5 aus Silikon. Der Federkörper 5 umfasst vier Federflächen 12 sowie vier Durchführungen 9 für die Stützstäbe 7. Analog zu den Halterungen 8 weisen auch die Durchführungen 9 eine innere Profilierung auf, in die das Profil der eingeführten Stützstäbe 7 derart eingreift, dass die Durchführungen 9 und die Stützstäbe 7 verdrehsicher miteinander verbunden sind.

Die Länge der vier Federflächen 12 entspricht im Wesentlichen dem durch die Länge der Stützstäbe 7 vorgegebenen Abstand der beiden Stirnteile 6 voneinander, während die Breite der Federflächen 12 jeweils etwas größer ist als der direkte Abstand der beiden Stützstäbe 7, zwischen denen sich die jeweilige Federfläche 12 erstreckt. Aus diesem Grund sind die Federflächen 12 zwischen den Stützstäben 7 nicht stramm gespannt sondern weisen eine Krümmung auf. Dabei sind zwei einander gegenüberliegende Federflächen 12, in der Darstellung der Figur 1 die rechte sowie die nicht sichtbare linke Federfläche 12, konkav nach innen gekrümmt, während die beiden übrigen, gemäß Figur 1 die obere und die ebenfalls nicht sichtbare untere Federfläche 12, konvex nach außen gekrümmt sind. Die beiden konkav gekrümmten Federflächen 12 werden im Folgenden auch als Primärflächen bezeichnet, die beiden konvex gekrümmten Federflächen 12 als Sekundärflächen.

Sowohl die Primärflächen als auch die Sekundärflächen liegen an der Außenwand der Volumenkammer 2 an und deformieren den Querschnitt der Volumenkammer 2, so dass dieser eine knochenförmige Gestalt aufweist, was andeutungsweise in Figur 4 zu erkennen ist. Die Federkräfte der Sekundärflächen können sehr klein gegenüber denen der Primärflächen sein. Wichtige Funktionen der Sekundärflächen sind die Führung und Halterung der Volumenkammer und die gezielte Störung bzw. Deformation des Druckbogens, den die Primärflächen aufbauen, bei Bluthochdruck, um das sprunghafte Ausweichen der Primärflächen auszulösen.

Das in den Figuren dargestellte Ausführungsbeispiel des Ausgleichsgefäß 1 weist eine Länge L von 112 mm, eine Breite B von 43 mm sowie eine Höhe H von 52 mm auf, vgl. Figuren 3 und 4.

Wird das erfindungsgemäße Ausgleichsgefäß 1 nun in der oben beschriebenen Weise an ein natürliches Blutgefäß angeschlossen, so zeigt sich in Abhängigkeit von dem herrschenden Blutdruck ein unterschiedliches Verhalten. In einem unteren Druckbereich unterhalb eines Druckschwellenwertes von 100 mmHg verhält sich das Ausgleichgefäß 1 im Wesentlichen wie ein natürliches Blutgefäß. Es ist also in der Lage, den oben beschriebenen Windkesseleffekt nachzubilden und lässt somit geringe Volumenänderungen der Volumenkammer 2 zu, diese werden jedoch durch den mit dem Rahmen 4 zusammenwirkenden und von außen an der Volumenkammer 2 anliegenden Federkörper 5 auf maximal 10 cm³ begrenzt.

Übersteigt der Blutdruck hingegen den Druckschwellenwert, so ist der Druck auf die Wand der Volumenkammer 2 und damit auf die an der Volumenkammer 2 anliegenden Federflächen 12 so groß, dass die beiden konkav gekrümmten Primärflächen sprunghaft nach außen ausweichen und in einen Zustand mit konvexer Krümmung übergehen. Entsprechend verändert sich der Querschnitt der Volumenkammer 2 von der im unteren Druckbereich vorliegenden Knochenform sprunghaft zu einer eher kreisförmigen bzw. elliptischen Form, wobei sich der Federkörper 5 im Bereich der Stützstäbe 7 aufgrund der verdrehsicheren Verbindung zwischen Federkörper 5 und Stützstäben 7 nicht verdreht. Durch die Querschnittsänderung vergrößert sich das Volumen der Volumenkammer 2 schlagartig um weitere ca. 20 cm³, wodurch der Blutdruck absinkt. Blutdruckspitzen von beispielsweise etwa 150 mmHg lassen sich auf diese Weise auf etwa 115 mmHg dämpfen. Die gesamte Volumenänderung der Volumenkammer 2 beträgt 20 bis 40 cm³, vorzugsweise etwa 30 cm³.

Es wird somit gewährleistet, dass das Ausgleichsgefäß 1 in Druckbereichen, die einem normalen Blutdruck entsprechen, keine über den natürlichen Windkesseleffekt hinausgehende Wirkung zeigt, während hoher Blutdruck wirksam gesenkt werden kann. Vorzugsweise wird das erfindungsgemäße Ausgleichsgefäß 1 bei Personen mit chronischem Bluthochdruck eingesetzt, bei denen das Ausgleichsgefäß 1 in der oben beschriebenen Weise permanent arbeitet. Aufgrund seiner Anpassungsfähigkeit an unterschiedliche Blutdrücke kann es jedoch grundsätzlich auch bei einem Personenkreis zum Einsatz kommen, der lediglich situationsabhängig unter erhöhtem Blutdruck leidet, ansonsten aber Blutdruckwerte im Normalbereich aufweist. Diese Flexibilität stellt einen großen Vorteil des erfindungsgemäßen Ausgleichsgefäßes 1 gegenüber einer medikamentösen Therapie dar.

Durch die verdrehsichere Verbindung der Stützstäbe 7 mit den Halterungen 8 der Stirnteile 6 und insbesondere mit den Durchführungen 9 des Federkörpers 5 wird der Effekt des plötzlichen Aufweitens der Volumenkammer 2 unterstützt. Durch die verdrehsichere Verbindung wird verhindert, dass die Federflächen 12 bei allmählich ansteigendem Druck in der Volumenkammer 2 durch Verdrehung der Durchführungen 9 gegenüber den Stützstäben 7 bereits nach außen ausweichen und sich so graduell an die veränderten Druckverhältnisse anpassen. Vielmehr sind die Federflächen 12 an ihren Längsseiten fest in den als Widerlager fungierenden Rahmen 4 eingespannt und ein Ausweichen der Federflächen 12 ist nur sprunghaft bei Überschreiten des Druckschwellenwertes möglich. Im Querschnitt betrachtet zeigen die Primärflächen damit ein ähnliches Verhalten wie ein eingespannter Knickstab. Sinkt der Blutdruck in der Folge wieder unter den Druckschwellenwert, so springen die Primärflächen wieder in ihre Ausgangsstellung zurück.

Die Stützstäbe 7 sind vorzugsweise biege- und torsionssteif ausgeführt. Die immer noch vorhandene Elastizität der Stützstäbe 7 beeinflusst das Federverhalten des Federkörpers 5.

Das konkrete Federverhalten der Primärflächen und damit der Druckschwellenwert lassen sich beispielsweise über die Dicke der Federflächen 12 und/oder über ein Rillenprofil 11 in den Primärflächen einstellen, wodurch die Federflächen 12 mehr oder weniger nachgiebig werden. Auch die konvex gekrümmten Sekundärflächen sowie die konkrete Dimensionierung von Primär- und Sekundärflächen haben einen Einfluss auf das Schaltverhalten der Primärflächen.

Mit Hilfe des erfindungsgemäßen Ausgleichsgefäßes lässt sich Bluthochdruck wirksam senken, und dies im Allgemeinen ohne dass eine zusätzliche Medikamenteneinnahme erforderlich wäre.

## Patentansprüche

1. Ausgleichsgefäß (1) zur Beeinflussung des Blutdrucks, umfassend eine Volumenkammer (2) mit Anschlussmitteln (3) zum Anschluss der Volumenkammer (2) an ein natürliches kardiovaskuläres System, wobei durch eine Druckänderung in dem kardiovaskulären System eine Volumenänderung der Volumenkammer (2) bewirkbar ist, sowie Anpassungsmittel, welche die Volumenänderung der Volumenkammer (2) in einem unteren Druckbereich unterhalb eines mindestens 100 mmHg betragenden Druckschwellenwertes auf maximal 10 cm³ begrenzen und welche in einem oberen Druckbereich zwischen dem Druckschwellenwert und 150 mmHg eine Volumenänderung der Volumenkammer (2) von mindestens 10 cm³ bewirken, **dadurch gekennzeichnet, dass** die Anpassungsmittel einen Rahmen (4) sowie mindestens einen mit diesem zusammenwirkenden Federkörper (5) umfassen, wobei Rahmen (4) und Federkörper (5) außerhalb der Volumenkammer (2) angeordnet sind und wobei der Rahmen (4) zwei Stirnteile (6) sowie mindestens drei die beiden Stirnteile (6) verbindende Stützstäbe (7) aufweist, zwischen denen sich der mindestens eine Federkörper (5) erstreckt.

2. Ausgleichsgefäß (1) nach Anspruch 1, **dadurch gekennzeichnet, dass** die Stirnteile (6) Halterungen (8) für die Aufnahme der Stützstäbe (7) aufweisen, in welchen die Stützstäbe (7) verdrehsicher gelagert sind.

3. Ausgleichsgefäß (1) nach Anspruch 1 oder 2, **dadurch gekennzeichnet, dass** der Federkörper (5) Durchführungen (9) für die Stützstäbe (7) aufweist.

4. Ausgleichsgefäß (1) nach Anspruch 3, **dadurch gekennzeichnet, dass** die Durchführungen (9) verdrehsicher mit den Stützstäben (7) verbunden sind.

5. Ausgleichsgefäß (1) nach einem der Ansprüche 1 bis 4, **dadurch gekennzeichnet, dass** der Federkörper (5) aus Silikon gefertigt ist.

6. Ausgleichsgefäß (1) nach einem der Ansprüche 1 bis 5, **dadurch gekennzeichnet, dass** der Federkörper (5) zumindest abschnittsweise an der Außenwand (10) der Volumenkammer (2) anliegt.

7. Ausgleichgefäß (1) nach einem der Ansprüche 1 bis 6, **dadurch gekennzeichnet, dass** der Federkörper (5) eine Profilierung (11) aufweist.

8. Ausgleichsgefäß (1) nach einem der Ansprüche 1 bis 7, **dadurch gekennzeichnet, dass** die Volumenkammer (2) aus Polyester gefertigt ist.

9. Ausgleichsgefäß (1) nach einem der Ansprüche 1 bis 8, **dadurch gekennzeichnet, dass** der Rahmen (4) vier die beiden Stirnteile (6) verbindende Stützstäbe (7) aufweist.

10. Ausgleichsgefäß (1) nach einem der Ansprüche 1 bis 9, **dadurch gekennzeichnet, dass** der Federkörper (5) Federflächen (12) umfasst, wobei sich jeweils eine Federfläche (12) zwischen zwei benachbarten Stützstäben (7) erstreckt.

11. Ausgleichsgefäß (1) nach Anspruch 10, **dadurch gekennzeichnet, dass** in dem unteren Druckbereich mindestens eine Federfläche (12) des Federkörpers (5) eine konkave Krümmung und mindestens eine Federfläche (12) des Federkörpers (5) eine konvexe Krümmung aufweist, während in dem oberen Druckbereich alle Federflächen (12) des Federkörpers (5) eine konvexe Krümmung aufweisen.

12. Ausgleichsgefäß (1) nach Anspruch 11, **dadurch gekennzeichnet, dass** der Federkörper (5) vier Federflächen (12) umfasst und dass in dem unteren Druckbereich zwei einander gegenüberliegende Federflächen (12) des Federkörpers (5) eine konkave Krümmung aufweisen und die beiden übrigen Federflächen (12) eine konvexe Krümmung aufweisen, während in dem oberen Druckbereich alle vier Federflächen (12) des Federkörpers (5) eine konvexe Krümmung aufweisen.

## Claims

1. A compensating vessel (1) for influencing blood pressure, comprising a volume chamber (2) with connecting means (3) for connecting the volume chamber (2) to a natural cardiovascular system, wherein a volume change of the volume chamber (2) can be caused by a pressure change in the cardiovascular system, and adaptation means, which limit the volume change of the volume chamber (2) in a lower pressure range below a pressure threshold value of at least 100 mmHg to a maximum of 10 cm³, and which cause a volume change of the volume chamber (2) of at least 10 cm³ in an upper pressure range between the pressure threshold value and 150 mmHg, **characterised in that** the adaptation means comprise a frame (4) and at least one resilient body (5) cooperating therewith, wherein the frame (4) and the resilient body (5) are disposed outside the volume chamber (2) and the frame (4) comprises two end parts (6) and at least three support bars (7), which connect the two end parts (6) and between which the at least one resilient body (5) extends.

2. The compensating vessel (1) according to claim 1, **characterised in that** the end parts (6) comprise brackets (8) for accommodating the support bars (7), in which the support bars (7) are mounted in a twist-proof manner.

3. The compensating vessel (1) according to claim 1 or 2, **characterised in that** the resilient body (5) comprises passages (9) for the support bars (7).

4. The compensating vessel (1) according to claim 3, **characterised in that** the passages (9) are connected in a twist-proof manner with the support bars (7).

5. The compensating vessel (1) according to any one of the claims 1 to 4, **characterised in that** the resilient body (5) is made from silicone.

6. The compensating vessel (1) according to any one of the claims 1 to 5, **characterised in that** the resilient body (5) rests at least in some sections against the outer wall (10) of the volume chamber (2).

7. The compensating vessel (1) according to any one of the claims 1 to 6, **characterised In that** the resilient body (5) has a profile (11).

8. The compensating vessel (1) according to any one of the claims 1 to 7, **characterised in that** the volume chamber (2) is made from polyester.

9. The compensating vessel (1) according to any one of the claims 1 to 8, **characterised in that** the frame (4) comprises four support bars (7) connecting the two end parts (6).

10. The compensating vessel (1) according to any one of the claims 1 to 9, **characterised in that** the resilient body (5) comprises resilient surfaces (12), with one resilient surface (12), respectively, extending between two adjacent support bars (7).

11. The compensating vessel (1) according to claim 10, **characterised in that**, in the lower pressure range, at least one resilient surface (12) of the resilient body (5) has a concave curvature and at least one resilient surface (12) of the resilient body (5) has a convex curvature, whereas, in the upper pressure range, all resilient surfaces (12) of the resilient body (5) have a convex curvature.

12. The compensating vessel (1) according to claim 11, **characterised in that** the resilient body (5) comprises four resilient surfaces (12) and that, in the lower pressure range, two resilient surfaces (12) of the resilient body (5) that are opposite from each other have a concave curvature and the two other resilient surfaces (12) have a convex curvature, whereas, in the upper pressure range, all four resilient surfaces (12) of the resilient body (5) have a convex curvature.

## Revendications

1. Tube de compensation (1) pour influencer la pression sanguine, comprenant une chambre de volume (2) avec des connexions (3) reliant la chambre de volume (2) à un système cardiovasculaire naturel, dans lequel une variation de la pression dans le système cardiovasculaire peut provoquer une variation du volume de la chambre de volume (2), ainsi que des moyens d'adaptation qui limitent la variation de volume de la chambre de volume (2) à 10 cm³ au maximum dans un domaine de plus basses pressions inférieures à une valeur seuil d'au moins 100 mmHg, et qui induisent dans un domaine de pressions plus élevées situées entre la valeur seuil et 150 mmHg, une variation de volume de la chambre de volume (2) d'au moins 10 cm³, **caractérisé en ce que** les moyens d'adaptation comprennent un cadre (4) lié à au moins un moyen de rappel (5), où le cadre (4) et les moyens de rappel (5) sont positionnés en dehors de la chambre de volume (2) et où le cadre (4) comporte deux parties frontales (6) ainsi qu'au moins trois barreaux de maintien (7) liant les parties frontales (6) entre elles, entre lesquels s'étirent les au moins un moyen de rappel (5).

2. Tube de compensation (1) selon la revendication 1, **caractérisé en ce que** les parties frontales (6) comprennent des fixations (8) pour accueillir des barreaux de maintien (7), de manière à empêcher la rotation des barreaux de maintien (7).

3. Tube de compensation (1) selon la revendication 1 ou 2, **caractérisé en ce que** le moyen de rappel (5) comprend des passages (9) pour les barreaux de maintien (7).

4. Tube de compensation (1) selon la revendication 3, **caractérisé en ce que** les passages (9) sont reliés aux barreaux de maintien (7) de manière à empêcher la rotation de l'ensemble.

5. Tube de compensation (1) selon l'une quelconque des revendications 1 à 4, **caractérisé en ce que** le moyen de rappel (5) est fait de silicone.

6. Tube de compensation (1) selon l'une quelconque des revendications 1 à 5, **caractérisé en ce que** le moyen de rappel (5) est au moins en partie ajusté aux parois extérieures (10) de la chambre de volume (2).

7. Tube de compensation (1) selon l'une quelconque des revendications 1 à 6, **caractérisé en ce que** le moyen de rappel (5) comprend un profilage (11).

8. Tube de compensation (1) selon l'une quelconque des revendications 1 à 7, **caractérisé en ce que** la chambre de volume (2) est faite de polyester.

9. Tube de compensation (1) selon l'une quelconque des revendications 1 à 8, **caractérisé en ce que** le cadre (4) comporte quatre barreaux de maintien (7) liant les parties frontales (6) entre elles.

10. Tube de compensation (1) selon l'une quelconque des revendications 1 à 9, **caractérisé en ce que** le moyen de rappel (5) comprend des surfaces de rappel (12) où une surface de rappel (12) s'étire systématiquement entre deux barreaux de maintien (7) voisins.

11. Tube de compensation (1) selon la revendication 10, dans lequel dans le domaine de plus basses pressions, au moins une surface de rappel (12) du moyen de rappel (5) présente une courbe concave et au moins une surface de rappel (12) du moyen de rappel (5) présente une courbe convexe, alors que dans le domaine de pressions plus élevées toutes les surfaces de rappel (12) du moyen de rappel (5) présentent une courbe convexe.

12. Tube de compensation (1) selon la revendication 11, dans lequel le moyen de rappel (5) comprend quatre surfaces de rappel (12), et où dans le domaine de plus basses pressions, deux surfaces de rappel (12) du moyen de rappel (5) opposées forment une courbe concave, les deux autres surfaces de rappel (12) formant une courbe convexe, alors que dans le domaine de pressions plus élevées, toutes les quatre surfaces de rappel (12) du moyen de rappel (5) forment une courbe convexe.
